# EUROPEAN PATENT SPECIFICATION

(11) **EP 0 614 880 B1**
(45) Date of publication and mention of the grant of the patent: **02.05.1997**
(21) Application number: 94107630.9
(22) Date of filing: 09.10.1990
(51) Int. Cl.: C07C 209/68, B01J 31/14

(54) **Ortho-alkylation of aromatic amines**
Ortho-alkylierung von aromatischen Aminen
Alkylation en position ortho d'amine aromatiques

(30) Priority: 10.10.1989 US 419059
(43) Date of publication of application: 14.09.1994
(62) Divisional of application: 90119354.0
(73) Proprietor: THE DOW CHEMICAL COMPANY, Midland, Michigan 48640 (US)
(72) Inventor: Guo-Shuh, John Lee, Midland, Michigan 48640 (US); Durvasula, V. Rao, Lake Jackson, Texas 77566 (US); Hartwell, Georg E., Midland, Michigan 48640 (US); Anderson, Kirk D., Texas 77566 (US); Moreno, Louis N., Lake Jackson, Texas 77566 (US); Shah, Nirad N., Lake Jackson, Texas 77566 (US)
(74) Representative: Huber, Bernhard, Dipl.-Chem.

(56) References cited:
- US-A- 4 760 185

## Description

The present invention relates to a process for the alkylation of aromatic amines, particularly the ortho-alkylation of aromatic diamines.

Processes for the preparation of ortho-alkylated aromatic amines from alkenes are known in the art. In one process, aluminum is dissolved in aniline to form aluminum anilide which serves as a catalyst for the alkylation of the aniline or of other aromatic amines. Such a process is taught in U. S. Patent 3,649,693 to Napolitano. Napolitano teaches that the alkylation itself is conducted at significantly elevated temperatures and pressures. Various other references have taught that the addition of mercury chloride, various Friedel Crafts catalysts, iodine or iodine compounds are helpful in improving the efficiency of the reaction.

U. S. Patent 4,760,185 to Becker teaches heating a diamine with an aluminum/zinc alloy and aluminum chloride in the absence of aniline until the evolution of hydrogen is complete.

According to this U.S. Patent it is critical to use an Al/Zn alloy and other aluminum alloys are held to be unsuitable for the production of an ortho-alkylation catalyst. This Al/Zn catalyst is then reacted with a lower alkene at significantly elevated pressure and temperature to form the alkylated phenylenediamine.

The methods known for the ortho-alkylation of aromatic diamines require substantially elevated reaction pressures and temperatures; require the presence of environmentally questionable substances such as mercury chloride; require expensive catalysts or require some combination of these factors. Thus what is needed is a method for the ortho-alkylation of aromatic amines which utilizes less expensive, less toxic catalysts and which utilizes relatively mild alkylation conditions.

The present invention involves a catalyst useful in a process for the ortho-alkylation of aromatic amines wherein the catalyst is prepared by heating an amine with
(1) an aluminum/magnesium alloy having a magnesium content from 0.5 to 30 weight percent;
(2) a Friedel Crafts catalyst selected from aluminum chloride, SnCl₄, SiCl₄, ZrI₄, FeCl₃ and BCl₃;
(3) zinc; and
(4) optionally, a solvent.
The completion of catalyst formation is indicated by the completion of the evolution of hydrogen. The present invention also involves the catalyst prepared by this method.

This invention also involves a process for the ortho-alkylation of aromatic amines wherein all or a portion of the catalyst prepared as described above is reacted, optionally with the addition of a second aromatic amine which may be the same as or different than the first aromatic amine, with an alkene at a temperature of at least 250°C and no greater than 350°C and a pressure of at least 1,800 kPascals and no greater than 21,600 kPascals under reaction conditions sufficient to produce the ortho-alkylated aromatic amines.

The process of the present invention results in the relatively rapid formation of the ortho-alkylated aromatic amines with high yields and selectivities.

The alkylated amines prepared by the process of this invention have a variety of uses. For example, alkylated phenylenediamines prepared by the process of this invention have a broad range of uses including use as antiknock agents in gasoline; as intermediates in the dye industry and as amine extenders in reaction injection molding.

Aromatic amines useful in the preparation of the catalysts of this invention include those compounds having at least one aromatic ring and at least one amine substituent directly bonded to the aromatic ring or rings.

In a preferred embodiment, aromatic diamines are useful in the preparation of the catalysts of this invention. Such preferred aromatic diamines correspond to the following formulas: 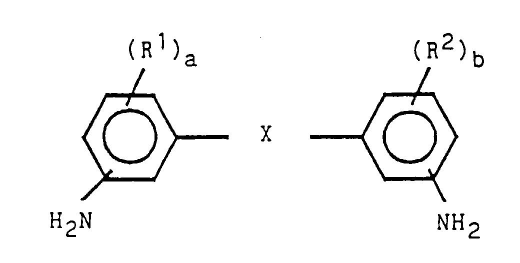 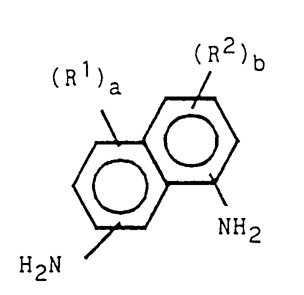 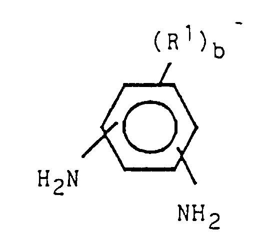 wherein X is a covalent bond, oxygen, sulfur or --CCR³R⁴)n-- wherein R³ and R⁴ are separately in each occurrence hydrogen or lower alkyl and n is 1 to 3; R¹ and R² are independently in each occurrence substituted or unsubstituted organic moieties such as alkyl, cycloalkyl and aralkyl moieties; and a and b are independently from zero to three. It is more preferred that R¹ and R² are independently in each occurrence substituted or unsubstituted C₁₋₈ organic moieties.

Non-limiting examples of preferred aromatic diamines useful in catalyst preparation include phenylenediamines such as m-phenylenediamine itself or substituted m-phenylenediamines. Useful substituents include C₁₋₈ organic moieties such as alkyl, cycloalkyl and aralkyl moieties. It is preferred that the substituents are C₁₋₃ alkyl groups. Non-limiting examples of substituted m-phenylenediamines useful in the catalyst preparation by the practice of this invention include 2,4-toluenediamine, 2,6-toluenediamine, 1-ethyl-2,4-phenylenediamine, 1-ethyl-2,6-phenylenediamine, 1-propyl-2,4-phenylenediamine, 1,3-dimethyl-4,6-phenylenediamine, 3,5-diethyl-2,4-toluenediamine, 3,5-diethyl-2,6-toluenediamine, 1-benzyl-2,4-phenylenediamine and mixtures thereof. The use of 2,4-toluenediamine, 2,6-toluenediamine, 3,5-diethyl-2,4-toluenediamine, 3,5-diethyl-2,6-toluenediamine and mixtures thereof is more preferred in the catalyst preparation process.

Aromatic monoamines such as aniline and substituted aniline are also useful in the preparation of the catalysts of the present invention.

Aluminum/magnesium alloys useful in the preparation of the catalysts useful in the practice of the present invention are those having a magnesium content of 0.5 weight percent to 30 weight percent and a corresponding aluminum content of 99.5 weight percent to 70 weight percent. It is preferred that the magnesium content is 2 weight percent to 10 weight percent. It is preferred to use commercially available aluminum/magnesium alloys such as Aluminum 5083, 5182, 5252, 6009, 6010, 6061, 6063 and 6201. As is recognized by those skilled in the art, aluminum/magnesium alloys may contain small amounts of various other metals such as silicon, copper and manganese.

A Friedel Crafts catalyst, preferably aluminum chloride, is used in the preparation of the catalysts of the present invention. In addition to aluminum chloride, SnCl₄, SiCl₄, ZrI₄, FeCl₃ and BCl₃ are useful in the practice of this invention. The use of aluminum chloride is preferred. The use of certain other salts, such as zinc chloride, is not preferred in the practice of the present invention.

The amount of aluminum chloride or other salt used in the preparation of the catalyst is that amount which will result in the formation of a catalyst which will result in the production of the desired alkylated amine. When aluminum chloride is used, the amount is preferably that amount of aluminum chloride which will result in a ratio of chlorine to aluminum atoms being in the range of 3:1 to 1:5; preferably 2:1 to 1:2; and most preferably about 1:1.

Zinc is used in the formation of the catalyst in the practice of this invention. Various forms of zinc are useful such as metallic zinc powder and dialkyl zinc. Any size of zinc particle which will function in the formation of the catalysts of this invention is useful. It is preferred that particle size not be too small due to problems associated with handling zinc dust. It is also preferred that particle size not be too large due to the very slow catalyst formation observed using large particles. It is preferred that the zinc be used in a particle size of from 75 microns in diameter to 850 microns in diameter. It is more preferred that the particles have a size range of from 90 to 300 microns in diameter, even more preferably from 100 to 200 microns in diameter and most preferably 125 to 175 microns.

The catalyst preparation of the present invention may be conducted neat or a solvent may be used. In some instances, a solvent/reactant may be used. In other instances, a solvent which is inert to the reaction may be used.

For purposes of the present invention, a solvent/reactant is a liquid aromatic amine capable of forming a catalyst system with aluminum, which also acts as a solvent. For example, in one embodiment of the present invention, the aromatic amine used in the preparation of the catalyst comprises diethyltoluenediamine which is a liquid in which at least some the various components used in catalyst preparation are soluble and thus acts as a solvent. Without wishing to be bound by any theory, it is believed that a portion of the diethyltoluenediamine present also appears to interact in some manner with the aluminum and other metals present to form the catalyst system which is itself soluble in the diethyltoluenediamine. The phrase "catalyst system" is used to describe the catalytic species that forms from the aromatic amine, aluminum and such other metals as are present. Non-limiting examples of solvent/reactants useful in the preparation of the catalysts of this invention include diethyltoluenediamine and aniline, including substituted anilines.

Inert solvents useful in the process of this invention are those solvents having good solubility for the catalyst system as described above, aluminum chloride and aromatic amines and also having good chemical and thermal stability. Such solvents include aromatic ether solvents which may be exemplified by phenoxy biphenyl and diphenyl ether; and alkyl polyaromatic solvents which may be exemplified terphenyl and diisopropylbiphenyl.

The catalyst is preferably formed by mixing an amine and solvent in a ratio ranging from 1:9 to 9:1 of amine to solvent by weight. It is more preferred that the amine to solvent ratio is greater than about 1:1. Alternatively, the amine is used without a solvent or a solvent/reactant is used. The aluminum alloy is preferably used in an amount such that there are at least 1 part and no greater than 10 parts of aluminum alloy per 100 parts of the amine. It is more preferred that there are 2 to 6 parts by weight of aluminum alloy per 100 parts by weight of diamine. The zinc is preferably used in an amount to provide at least 0.05 and no greater than 10 parts zinc by weight per 100 parts of amine It is more preferred that there is 0.1 to 2.5 parts by weight of zinc per 100 parts by weight of amine. Aluminum chloride is preferably used in an amount to provide a chlorine to aluminum atom ratio of about 1:1.

The slurry resulting from the above mixture is heated with the evolution of hydrogen until the metals have dissolved. The completion of catalyst formation is indicated by the cessation of hydrogen evolution. After venting the hydrogen, the resulting catalyst is used in the alkylation process in an amount sufficient to provide preferably at least 1 and no greater than 10 weight percent metals based on the weight of the amine to be alkylated. Metals in this context refer to either the aluminum alloy or the mixture of aluminum alloy and zinc metals. It is preferred to use sufficient catalyst to provide at least 2 and no greater than 6 weight percent metals.

The alkylation process of the present invention is useful in the ortho-alkylation of the amine used in the catalyst preparation and in the ortho-alkylation of amines differing from the amine used in the catalyst preparation. In the former situation, catalyst preparation and alkylation may take place in a single reactor without isolation of the catalyst itself, although separate reactors may be used. In the latter situation, the catalyst is prepared in one step and may be stored or transported as necessary and then used in a separate alkylation process. For example, toluenediamine may be used in the preparation of the catalyst and also be the aromatic amine which is alkylated. In contrast, diethyltoluenediamine may be used in the catalyst preparation and toluenediamine used as the aromatic amine to be alkylated.

As discussed above, the aromatic amine to be alkylated may be the same amine used in the preparation of the catalyst or may be a different amine. The aromatic amines which may be alkylated by the process of this invention are the same as those used in catalyst preparation with the exception that the amines to be alkylated are not substituted in the positions ortho to the amine group(s). In a preferred embodiment, toluenediamine is ortho-alkylated to form diethyltoluenediamine.

The alkenes useful in this invention are alkenes containing from two to eighteen carbon atoms. The alkenes may be unsubstituted or may contain inert substituents and may contain single or multiple unsaturations. It is preferred that the alkenes contain from two to twelve carbon atoms and more preferred that they contain from two to six carbon atoms. It is most preferred to use alkenes containing from two to four carbon atoms. Non-limiting examples of alkenes useful in the alkylation reaction of this invention include ethene, propene and butene. It is particularly preferred to use ethene.

One skilled in the art will recognize that both the alkylation reaction and the catalyst preparation and their combination may be conducted in a batch or continuous manner. As discussed above, the catalyst preparation may be conducted separately from the alkylation reaction in that the catalyst is prepared and then stored and/or transported prior to being used in the alkylation reaction. Alternatively, the catalyst may be prepared, but not isolated or collected prior to being used in the alkylation reaction. Those skilled in the art will recognize that choice of reactors will depend in part on whether the catalyst preparation and alkylation reactions take place in the same reactor. The reactor in which the catalyst is prepared must be designed to withstand hydrogen production and should be free of metals known to be detrimental to the formation of the catalyst system. An example of such a reactor is a glass-lined reactor. The reactor used in the alkylation process should be inert and capable of withstanding elevated pressures and temperatures. Examples of reactors useful in the alkylation process include those constructed of carbon steel, titanium and zirconium. Clearly, those processes in which the catalyst is prepared and the alkylation reaction occurs in the same reactor will require a reactor which meets the criteria of both.

The choice of conditions useful in the alkylation reaction of the present invention will depend, in part, on the choice of alkene used as an alkylating agent. For example, one skilled in the art will recognize that alkylation pressures and temperatures will vary depending on the alkylating agent. One skilled in the art will also recognize that the choice of other reaction parameters, whether the alkylation reaction should be done in a batch or a continuous manner and similar decisions will be made based on the particular requirements of a given process. It will also be recognized that one skilled in the art understands the dangers of working at high presssures and that selections of temperatures and pressures will be made in accordance with safe engineering practices.

In this invention temperatures for the alkylation process are at least 250°C and no greater than 350°C. If the alkylene used is ethene, it is more preferred that the ethylation process be conducted at temperatures of at least 270°C and no greater than 330°C. Ethene pressures preferred in alkylation process range from at least 1800 kPascals up to 21,600 kPascals. Due to the expense of operating high pressure equipment, lower pressures are often preferred for commercial reasons. In a particularly preferred embodiment, the ethylation step is conducted at pressures of at least 1800 kPascals to no greater than 10,800 kPascals, more preferably no greater than 7200 kPascals, and most preferably no greater than 4800 kPascals.

## Claims

1. A method of preparing a catalyst comprising heating an amine with
(1) an aluminum/magnesium alloy having a magnesium content from 0.5 to 30 weight percent;
(2) a Friedel Crafts catalyst selected from aluminum chloride, SnCl₄, SiCl₄, ZrI₄, FeCl₃ and BCl₃;
(3) zinc; and
(4) optionally, a solvent.

2. The method of Claim 1 wherein the amine is selected from toluenediamine, diethyltoluenediamine and mixtures thereof.

3. The method of Claims 1 or 2 wherein the Friedel Crafts catalyst is aluminum chloride.

4. The method of Claims 1, 2 or 3 wherein zinc is used and has an average particle diameter ranging from 75 microns to 850 microns.

5. A process for the preparation of ortho-alkylated amines comprising preparing a catalyst by a method according to claim 1 and subsequently reacting the catalyst with an alkene and, optionally, a second aromatic amine at a temperature of at least 250°C and no greater than 350°C and a pressure of at least 1800 kPascals and no greater than 21,600 kPascals under reaction conditions sufficient to produce the ortho-alkylated amines.

6. The process of Claim 5 wherein the first aromatic amine is selected from the group consisting of toluenediamine, diethyltoluenediamine and mixtures thereof.

7. The process of Claims 5 or 6 wherein the second aromatic amine is used and is toluenediamine.

8. The process of Claims 5, 6 or 7 wherein zinc is used in the preparation of the catalyst and has a particle diameter of from 75 to 850 microns.

9. The process of Claim 8 wherein the elevated pressure is no greater than 7200 kPascals.

10. A catalyst obtainable by heating an amine with
(1) an aluminum/magnesium alloy having a magnesium content from 0.5 to 30 weight percent;
(2) a Friedel Crafts catalyst selected from aluminum chloride, SnCl₄, SiCl₄, ZrI₄, FeCl₃ and BCl₃;
(3) zinc; and
(4) optionally, a solvent.

## Patentansprüche

1. Verfahren zur Herstellung eines Katalysators, umfassend das Erhitzen eines Amins mit
(1) einer Aluminium/Magnesium-Legierung mit einem Magnesiumgehalt von 0,5 bis 30 Gewichtsprozent;
(2) einem Friedel-Crafts-Katalysator, ausgewählt aus Aluminiumchlorid, SnCl₄, SiCl₄, ZrI₄, FeCl₃ und BCl₃;
(3) Zink; und
(4) gegebenenfalls einem Lösungsmittel.

2. Verfahren nach Anspruch 1, worin das Amin aus Toluoldiamin, Diethyltoluoldiamin und Gemischen davon ausgewählt wird.

3. Verfahren nach Anspruch 1 oder 2, worin der Friedel-Crafts-Katalysator Aluminiumchlorid ist.

4. Verfahren nach Anspruch 1, 2 oder 3, worin Zink verwendet wird und einen mittleren Teilchendurchmesser im Bereich von 75 Mikrometer bis 850 Mikrometer hat.

5. Verfahren zur Herstellung ortho-alkylierter Amine, umfassend die Herstellung eines Katalysators durch ein Verfahren nach Anspruch 1 und nachfolgendes Umsetzen des Katalysators mit einem Alken und gegebenenfalls einem zweiten aromatischen Amin, bei einer Temperatur von mindestens 250°C und nicht mehr als 350°C und einem Druck von mindestens 1800 kPascal und nicht mehr als 21.600 kPascal, unter Reaktionsbedingungen, die ausreichend sind, um die orthoalkylierten Amine zu bilden.

6. Verfahren nach Anspruch 5, worin das erste aromatische Amin ausgewählt wird aus der aus Toluoldiamin, Diethyltoluoldiamin und Gemischen davon bestehenden Gruppe.

7. Verfahren nach Anspruch 5 oder 6, worin das zweite aromatische Amin verwendet wird und Toluoldiamin ist.

8. Verfahren nach Anspruch 5, 6 oder 7, worin Zink bei der Herstellung des Katalysators verwendet wird und einen Teilchendurchmesser von 75 bis 850 Mikrometer hat.

9. Verfahren nach Anspruch 8, worin der erhöhte Druck nicht größer als 7200 kPascal ist.

10. Katalysator, erhältlich durch Erhitzen eines Amins mit
(1) einer Aluminium/Magnesium-Legierung mit einem Magnesiumgehalt von 0,5 bis 30 Gewichtsprozent;
(2) einem Friedel-Crafts-Katalysator, ausgwählt aus Aluminiumchlorid, SnCl₄, SiCl₄, ZrI₄, FeCl₃ und BCl₃;
(3) Zink; und
(4) gegebenenfalls einem Lösungsmittel.

## Revendications

1. Procédé pour préparer un catalyseur consistant à chauffer une amine avec
(1) un alliage aluminium/magnésium ayant une teneur en magnésium allant de 0,5 à 30 pourcent en poids;
(2) un catalyseur de Friedel et Crafts choisi parmi le chlorure d'aluminium, SnCl₄, SiCl₄, ZrI₄, FeCl₃ et BCl₃;
(3) du zinc; et
(4) éventuellement, un solvant.

2. Procédé selon la revendication 1, dans lequel l'amine est choisie parmi la toluènediamine, la diéthyltoluènediamine et leurs mélanges.

3. Procédé selon l'une des revendications 1 ou 2, dans lequel le catalyseur de Friedel Crafts est le chlorure d'aluminium.

4. Procédé selon l'une des revendications 1, 2 ou 3, dans lequel on utilise du zinc sous forme de particules ayant un diamètre moyen de 75 micromètres à 850 micromètres.

5. Procédé pour la préparation d'amines ortho-alkylées consistant à préparer un catalyseur à l'aide d'un procédé selon la revendication 1 et ultérieurement à faire réagir le catalyseur avec un alcène et, éventuellement, une deuxième amine aromatique à une température d'au moins 250°C et pas supérieure à 350°C et sous une pression d'au moins 1 800 kPa et pas supérieure à 21 600 kPa dans des conditions réactionnelles suffisantes pour produire les amines ortho-alkylées.

6. Procédé selon la revendication 5, dans lequel on choisit la première amine aromatique dans le groupe composé de la toluènediamine, de la diéthyltoluènediamine et de leurs mélanges.

7. Procédé selon l'une des revendications 5 ou 6, dans lequel on utilise la deuxième amine aromatique et elle est la toluènediamine.

8. Procédé selon l'une des revendications 5, 6 ou 7, dans lequel on utilise du zinc dans la préparation du catalyseur sous forme de particules ayant un diamètre de 75 à 850 micromètres.

9. Procédé selon la revendication 8, dans lequel la pression élevée n'est pas supérieure à 7 200 kPa.

10. Catalyseur susceptible d'être obtenu en chauffant une amine avec
(1) un alliage aluminium/magnésium ayant une teneur en magnésium allant de 0,5 à 30 pourcent en poids;
(2) un catalyseur de Friedel et Crafts choisi parmi le chlorure d'aluminium, SnCl₄, SiCl₄, ZrI₄, FeCl₃ et BCl₃;
(3) du zinc; et
(4) éventuellement, un solvant.
